# EUROPEAN PATENT SPECIFICATION

(11) **EP 2 609 865 B1**
(45) Date of publication and mention of the grant of the patent: **28.09.2022**
(21) Application number: 12173189.7
(22) Date of filing: 22.06.2012
(51) Int. Cl.: A61B 8/00, A61B 8/08

(54) **Method for providing body marker and ultrasound diagnostic apparatus therefor**
Verfahren zur Bereitstellung von Körpermarkern und Ultraschalldiagnosevorrichtung dafür
Procédé permettant de fournir un marqueur corporel et appareil de diagnostic à ultrasons associé

(30) Priority: 28.12.2011 KR 20110144984
(43) Date of publication of application: 03.07.2013
(73) Proprietor: Samsung Medison Co., Ltd., Kangwon-do 250-875 (KR)
(72) Inventor: Lee, Jun-kyo, 250-875 Gangwon-do (KR)
(74) Representative: Schmid, Wolfgang

(56) References cited:
- WO-A2-99/27839
- US-A1- 2001 035 871
- US-A1- 2003 114 741

## Description

### BACKGROUND OF THE INVENTION

### 1. Field of the Invention

The present invention relates to a method and an apparatus for providing a body marker by using an image of a testee.

### 2. Description of the Related Art

An ultrasound diagnostic device is a device for transmitting ultrasound signals from a surface of a target body toward a predetermined region inside the target body and acquiring tomograms of soft tissues or images of blood flow based on information of ultrasound signals reflected by tissues inside the target body.

The advantages of such an ultrasound diagnostic device are its small size, inexpensive price, and real-time result acquisition. Furthermore, an ultrasound diagnostic device is very safe and causes no radiation exposure. Therefore, an ultrasound diagnostic device is widely used together with other types of imaging diagnostic devices, such as X-ray devices, computerized tomography (CT) scanners, magnetic resonance image (MRI) devices, scintillation cameras, etc., for mediacal diagnosis and treatment.

Generally, an ultrasound diagnostic device operates in a brightness mode (B mode) wherein the intensities of ultrasound echo signals reflected by a target body are indicated via a brightness scale, a Doppler mode wherein an image of a moving target body is indicated via a spectrum by using the Doppler effect, a motion mode (M mode) wherein a motion of a target body at a constant location is indicated according to time, an elasticity mode wherein a difference between a reaction of a target body when the target body is compressed and a reaction of the target body when the target body is not compressed is indicated, and and a color mode image (C mode image) wherein a velocity of a moving target body is indicated via colors by using the Doppler effect. Furthermore, an ultrasound diagnostic device provides a user-selectable body marker.

US 2001/035871 A1 describes that in a system and a method for generating an image a first system acquires an image dataset from a subject and a second system obtains a video image of the subject. The positions of the first and second system are determined with a navigation system, with reference to which the position of the image dataset acquired with the first system and the position of the video image dataset can be determined in space.

WO 99/27839 A2 discloses a system for positioning and repositioning of a portion of a patient's body with respect to a treatment or imaging machine and includes multiple cameras to view the body and the machine. Index markers are identified and located by cameras in 3D space and are in a determinable relationship to analogous markers used during previous image scanning of the patient.

US 2003/114741 A1 relates to a device for projecting patient image data from radioscopic and/or tomographic imaging methods onto video images.

### SUMMARY OF THE INVENTION

According to an aspect of the present invention, there is provided a method of providing a body marker according to claim 1.

According to another aspect of the present invention, there is provided an ultrasound diagnostic device according to claim 6.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of a body marker providing apparatus according to an embodiment of the present invention;
FIG. 2 is a block diagram of a body marker providing apparatus according to another embodiment of the present invention;
FIG. 3 is a flowchart for describing a method of providing a body marker according to an embodiment of the present invention;
FIG. 4 is a flowchart for describing a method of generating a body marker by using a body model according to an embodiment of the present invention;
FIG. 5 is a flowchart for describing a method of displaying a body marker according to an embodiment of the present invention;
FIG. 6 is a flowchart for describing a method of automatically setting functions of a probe by using information regarding location of the probe according to an embodiment of the present invention; and
FIG. 7 is a diagram showing a body marker according to an embodiment of the present invention.

### DETAILED DESCRIPTION OF THE INVENTION

In addition, although the terms used in the present invention are selected from generally known and used terms, some of the terms mentioned in the description of the present invention have been selected by the applicant at his or her discretion, the detailed meanings of which are described in relevant parts of the description herein. Furthermore, it is required that the present invention is understood, not simply by the actual terms used but by the meaning of each term lying within.

In addition, unless explicitly described to the contrary, the word "comprise" and variations such as "comprises" or "comprising" will be understood to imply the inclusion of stated elements but not the exclusion of any other elements. In addition, the terms "-er", "-or", and "module" described in the specification mean units for processing at least one function and operation and can be implemented by hardware components or software components and combinations thereof.

Hereinafter, the term "ultrasound image" means an image of a target object that is obtained by using ultrasound waves. The target object may refer to a part of a human body. For example, a target object may include an internal organ, such as a liver, heart, uterus, brain, breast, stomach, etc., or a fetus.

Hereinafter, a "user" is a medical expert, e.g., a doctor, a nurse, a medical laboratory technician, a medical imaging expert, etc., but is not limited thereto.

Hereinafter, a "body marker" is a geometric symbol indicating a location to which ultrasound image is scanned or a target object. A general example of body markers may include a liver mark.

Hereinafter, the present invention will be described in detail by explaining preferred embodiments of the invention with reference to the attached drawings. Like reference numerals in the drawings denote like elements.

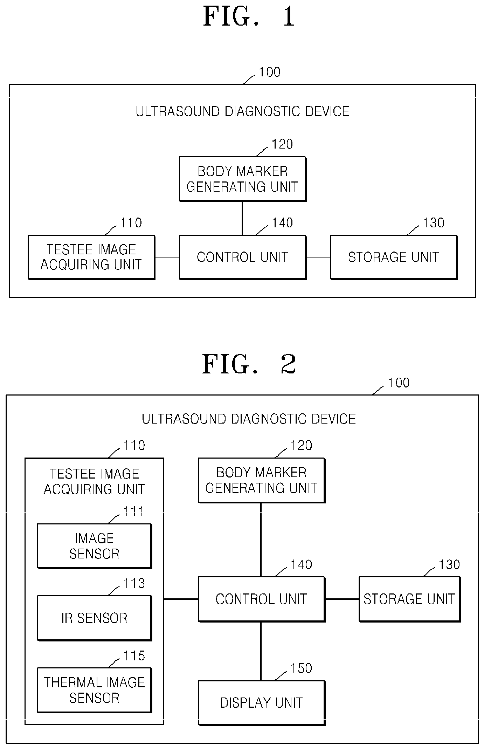

FIG. 1 is a block diagram of a body marker providing apparatus according to an embodiment of the present invention.

FIG. 2 is a block diagram of a body marker providing apparatus according to another embodiment of the present invention.

An ultrasound diagnostic device 100 according to an embodiment of the present invention is a device capable of acquiring an ultrasound image from a target body by using ultrasounds and providing a body marker related to the acquired ultrasound image to a user.

The ultrasound diagnostic device 100 according to an embodiment of the present invention may be embodied in various ways. For example, the ultrasound diagnostic device 100 may be embodied not only as a fixed terminal, but also as a mobile terminal. Examples of the mobile terminals may include a laptop computer, a personal digital assistant (PDA), and a tablet PC.

According to an embodiment of the present invention, the ultrasound diagnostic device 100 may include a probe (not shown), a testee image acquiring unit 110, a body marker generating unit 120, a storage unit 130, a control unit 140, and a display unit 150. However, not all of the above-stated components are striclty required components. The ultrasound diagnostic device 100 may be emobided by using less or more components than the above-stated components.

The probe (not shown) may transmit ultrasound signals to a target body. The probe may also receive ultrasound echo signals from the target body. The ultrasound diagnostic device 100 may generate ultrasound images with respect to the target body based on ultrasound echo signals received from the target body.

The testee image acquiring unit 110 may acquire an image of a testee. According to an embodiment of the present invention, if the ultrasound diagnostic device 100 generates an ultrasound image of a target body by using the probe, the testee image acquiring unit 110 may acquire an image of a testee including information regarding location of the probe.

The information regarding location of the probe refers to information regarding locations of the probe when an image of a testee is acquired. The information regarding location of the probe according to an embodiment of the present invention may include relative location of the probe in an image of a testee, an indication marker for indicating location of the probe, and coordinates of location of the probe.

According to an embodiment of the present invention, the testee image acquiring unit 110 may simultaneously acquire information regarding location of the probe and an image of a testee. According to another embodiment of the present invention, the testee image acquiring unit 110 may acquire information regarding location of the probe and an iamge of a testee separately.

The testee image acquiring unit 110 may include an image sensor 111, an infrared ray (IR) sensor 113, and a thermal image sensor 115. In other words, according to an embodiment of the present invention, the testee image acquiring unit 110 may be embodied as at least one of an imaging camera including the image sensor 111, an IR camera including the IR sensor 113, and a thermal imaging camera including the thermal image sensor 115.

The testee image acquiring unit 110 according to an embodiment of the present invention may be arranged inside or outside the ultrasound diagnostic device 100.

The body marker generating unit 120 may generate a body marker based on an image of a testee including the information regarding location of the probe. The body marker generating unit 120 may extract frame information from the image of the testee. The body marker generating unit 120 according to an embodiment of the present invention may generate a body marker with respect to a target body of the testee by using the extracted frame information and the information regarding location of the probe.

According to an embodiment of the present invention, the ultrasound diagnostic device 100 generates a body marker by using an actual image of a testee, and thus, the ultrasound diagnostic device 100 may present the target body of the testee clearly. Furthermore, the user's inconvenience in selecting a body marker may be reduced.

The body marker generating unit 120 may generate a body marker in the form of at least one from among a 2-dimensional image, a 3-dimensional, and a 4-dimensional.

According to an embodiment of the present invention, the body marker generating unit 120 may generate a body marker by using a pre-stored body model. The body marker generating unit 120 may refer to a pre-stored body model from a memory. The body marker generating unit 120 may compare an image of a testee to the pre-stored body model. Next, the body marker generating unit 120 may modify the pre-stored body model in correspondence to the image of the testee. The body marker generating unit 120 may generate a body marker based on the modified body model and the information regarding location of the probe.

The storage unit 130 may store a program for processes and controls of the control unit 140 and may temporarily store input/output data. For example, the storage unit 130 may map a body marker to an ultrasound image regarding a target body and store a mapped result. According to an embodiment of the present invention, either a single ultrasound image or a plurality of ultrasound images may be used.

An ultrasound image according to an embodiment of the present invention may include at least one of a brightness mode (B mode) image, a motion mode (M mode) image, an elasticity mode image, a 2-dimensional mode image, and a 3-dimensional mode image.

The storage unit 130 may store a body model. A body model according to an embodiment of the present invention may be used as a body marker instead of an image of a testee for protection of privacy.

The storage unit 130 may include at least one of various types of storage medium including a flash memory type storage medium, a hard disk type storage medium, a multimedia card micro type storage medium, a card type memory (e.g., a SD memory or a XD memory), a random access memory (RAM) a static RAM (SRAM), a read-only memory (ROM), an electrically erasable programmable read-only memory (EEPROM), a programmable ROM (PROM), a magnetic memory, a magnetic disk, and an optical disc. Furthermore, the ultrasound diagnostic device 100 may operate a web storage, which functions as the storage unit 130, on the Internet.

The control unit 140 generally controls overall operations of the ultrasound diagnostic device 100. The control unit 140 may control overall operations of the probe, the iamge acquring unit 110, the body marker generating unit 120, the storage unit 130, and the display unit 150.

The control unit 140 may extract a target body of the testee corresponding to the information regarding location of the probe. Next, the control unit 140 may set functions for diagnosing the extracted target body to the probe.

The display unit 150 may display information processed by the ultrasound diagnostic device 100. For example, the display unit 150 may display an ultrasound image and a body marker corresponding to the ultrasound image.

If the display unit 150 and a touch pad form a mutual layer structure that constitutes a touch screen, the display unit 150 may be used not only as an output device, but also as an input device. The display unit 150 may include at least one of a liquid crystal display, a thin film transistor-liquid crystal display, an organic light-emitting diode, a flexible display, and a 3-dimensional display.

Furthermore, according to configurations of the ultrasound diagnostic device 100, two or more display units 150 may be arranged. A touch screen may be configured to detect not only a touch input location and a touched area, but also a touch input pressure. Furthermore, the touch screen may be configured to detect not only a real-touch, but also a proximity touch.

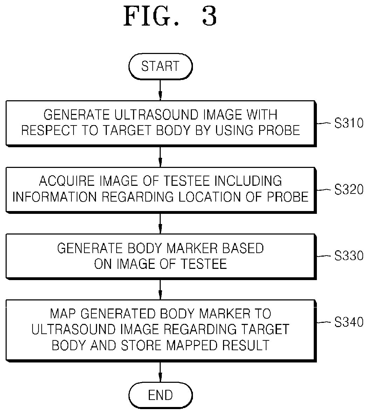

Hereinafter, a method of providing an accurate body marker regarding a target body will be dsecribed in detail with reference to FIG. 3.

FIG. 3 is a flowchart for describing a method of providing a body marker according to an embodiment of the present invention.

Referring to FIG. 3, the method of providing a body marker according to the present embodiment includes operations that are chronologically performed by the ultrasound diagnostic device 100 shown in FIG. 2. Therefore, even if omitted below, descriptions given above regarding the ultrasound diagnostic device 100 shown in FIGS. 1 and 2 may also be applied to the method of providing a body marker shown in FIG. 3.

The ultrasound diagnostic device 100 may generate an ultrasound image with respect to a target body by using a probe (operation S310). Here, according to an embodiment of the present invention, the ultrasound diagnostic device 100 may acquire an image of a testee including information regarding location of the probe as of generation of the ultrasound signal (operation S320).

For example, if the ultrasound diagnostic device 100 is to acquire an ultrasound image with respect to the abdomen of a testee, the probe may be located on the abdoment of the testee. Here, the ultrasound diagnostic device 100 may acquire an image of the testee including an image of the probe located on the abdoment of the testee.

According to an embodiment of the present invention, the ultrasound diagnostic device 100 may acquire an image of a testee by using at leaste one of an image sensor, an IR sensor, and a thermal image sensor.

According to an embodiment of the present invention, the ultrasound diagnostic device 100 may generate a body marker based on the image of the testee including the information regarding location of the probe (operation S330). In other words, according to an embodiment of the present invention, the ultrasound diagnostic device 100 may generate a body marker by using the information regarding location of the probe and the image of the testee.

For example, the ultrasound diagnostic device 100 may extract frame information from the image of the testee. The frame information accoring to an embodiment of the present invention may include an image regarding bones constituting and supporting a body or information regarding pose of the testee.

The ultrasound diagnostic device 100 may generate a body marker by using the extracted frame information and the information regarding location of the probe.

For example, if a testee A lies down on his/her back for ultrasound diagnosis, the ultrasound diagnostic device 100 may acquire an image of the testee A and extract frame information from an image of the testee A. Since the ultrasound diagnostic device 100 acquires images of the testee A while the testee A is lying down for ultrasound diagnosis, the image of the testee A may include an image of the probe.

The ultrasound diagnostic device 100 may generate a body marker for indicating a target body based on the extracted frame information and the information regarding location of the probe. The body marker generated by the ultrasound diagnostic device 100 may include an entire image of the probe or any of various icons indicating location of the probe. For example, the body marker may indicate location of the probe by using an arrow symbol (e.g., 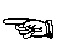 , ↑ , ▼ , etc.) or an emoticon (e.g., ★, ◆ , ×, ⊚ , etc).

Therefore, according to an embodiment of the present invention, the ultrasound diagnostic device 100 may generate a body marker in which location of the probe is indicated on a frame image of a testee, and thus the ultrasound diagnostic device 100 may indicate accurate location of the probe as of acquisition of an ultrasound image, that is, location of a target body.

According to an embodiment of the presetn invention, the ultrasound diagnostic device 100 may map the generated body marker to an ultrasound image regarding a target body and store amapped result (operation S340). According to an embodiment of the present invention, the ultrasound diagnostic device 100 may map a single body marker to a plurality of ultrasound image and store a mapped result.

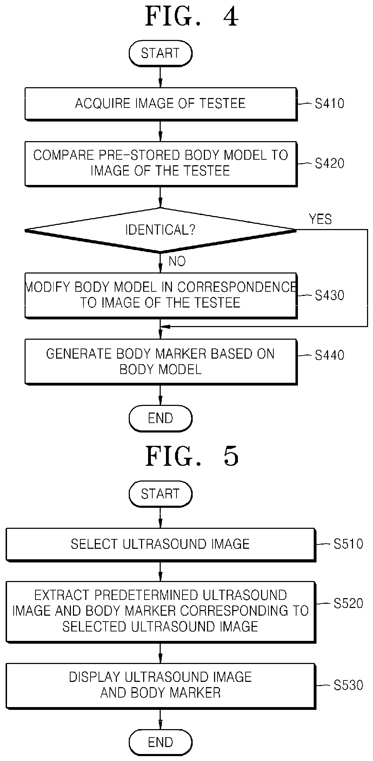

FIG. 4 is a flowchart for describing a method of generating a body marker by using a body model according to an embodiment of the present invention.

As shown in FIG. 4, according to an embodiment of the present invention, the ultrasound diagnostic device 100 may acquire an image of a testee (operation S410). The image of the testee may include information regarding location of the probe.

The ultrasound diagnostic device 100 may acquire a body model. A body model according to an embodiment of the present invention may be pre-stored in the storage unit 130.

The ultrasound diagnostic device 100 may compare the body model to the iamge of the testee (operation S420). For example, the ultrasound diagnostic device 100 may compare the image of the testee with respect to the size and pose of the body model.

According to an embodiment of the present invention, the ultrasound diagnostic device 100 may extract frame information from the image of the testee and compare the image of the testee to the body model based on the extracted frame information.

If the image of the testee is not identical to the body model, the ultrasound diagnostic device 100 may modify the body model in correspondence to the image of the testee (operation S430). According to an embodiment of the present invention, the ultrasound diagnostic device 100 may modify the body model based on the frame information extracted from the image of the testee.

For example, the image of the testee may show that the testee is lying down on his/her back, whereas the body model may be standing up. In this case, the ultrasound diagnostic device 100 may modify the body model to a laid-down pose based on the image of the testee.

If the image of the testee is identical to the body model, the ultrasound diagnostic device 100 may generate a body marker based on the body model and the information regarding location of the probe (operation S440). For example, the ultrasound diagnostic device 100 may fit size or pose of the body model to size or pose of the testee and apply information regarding location of the probe included in the image of the testee to the body model. In other words, the ultrasound diagnostic device 100 may generate a body marker by applying the information regarding location of the probe to the body model of which pose or size is fitted to the image of the testee.

According to an embodiment of the present invention, the ultrasound diagnostic device 100 may protect the privacy of a testee by generating a body marker by using a body marker that is fitted to an actual image of the testee. Furthermore, according to an embodiment of the present invention, the ultrasound diagnostic device 100 may generate a body marker in the form of a 3-dimensional image by using the body model.

FIG. 5 is a flowchart for describing a method of displaying a body marker according to an embodiment of the present invention.

As shown in FIG. 5, the ultrasound diagnostic device 100 may select a predetermined ultrasound image (operation S510). According to an embodiment of the present invention, the ultrasound diagnostic device 100 may select a predetermined image according to a user input. In this case, the ultrasound diagnostic device 100 may extract the predetermined ultrasound image and a body marker corresponding to the predetermined ultrasound image from the storage unit 130 (operation S520).

According to an embodiment of the present invention, the ultrasound diagnostic device 100 may display the ultrasound image and the body marker to a user (operation S530).

For example, if a user selects an ultrasound image as of March 5^{th}, 2011 with repsect to a patient A, the ultrasound diagnostic device 100 may extract the ultrasound image of the patient A as of March 5^{th}, 2011. Furthermore, the ultrasound diagnostic device 100 may extract a body marker regarding the ultrasound image of the patient A as of March 5^{th}, 2011 and display the ultrasound image of the patient A as of March 5^{th}, 2011 and the body marker regarding the ultrasound image of the patient A as of March 5^{th}, 2011 together to the user. In this case, the body marker may be indication of information regarding location of a probe on the image of the patient A.

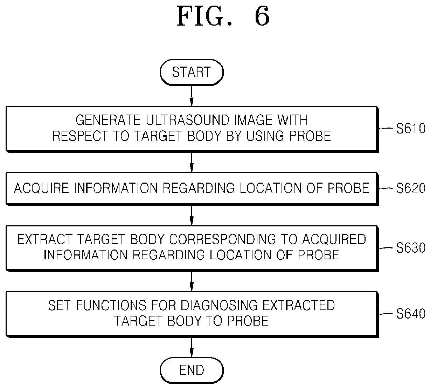

FIG. 6 is a flowchart for describing a method of automatically setting functions of a probe by using information regarding location of the probe according to an embodiment of the present invention.

As shown in FIG. 6, the ultrasound diagnostic device 100 may generate an ultrasound image with respect to a target body by using a probe (operation S610). Here, the ultrasound diagnostic device 100 may acquire information regarding location of the probe (operation S620). For example, the ultrasound diagnostic device 100 may acquire information regarding location of the probe on a testee by using an imaging camera.

The ultrasound diagnostic device 100 may extract a target body corresponding to the acquired information regarding location of the probe (operation S630). For example, if the probe is located on the head of the testee, the ultrasound diagnostic device 100 may extract 'brain' as the target body corresponding to the information regarding location of the probe. Furthermore, if the probe is located on the left chest of the testee, the ultrasound diagnostic device 100 may extract 'heart' as the target body corresponding to the information regarding location of the probe.

According to an embodiment of the present invention, the ultrasound diagnostic device 100 may set functions for diagnosing the extracted target body (operation S640). In other words, according to an embodiment of the present invention, the ultrasound diagnostic device 100 may automatically select default values for diagnosing a corresponding target body when the probe contacts the body of a testee.

For example, if the target body corresponding to the information regarding location of the probe is 'heart,' the ultrasound diagnostic device 100 may set functions (or modes and default values) for heart diagnosis to the probe.

Therefore, according to an embodiment of the present invention, even if a user does not separately adjusts default values for settting functions for dianosing a target body to a probe, the ultrasound diagnostic device 100 may automatically change settings of the probe according to location of the probe. Therefore, user convenience may be improved.

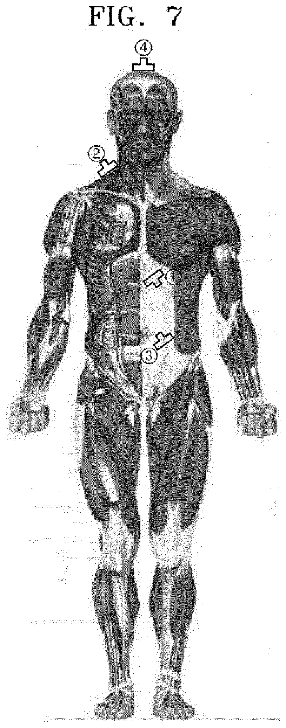

FIG. 7 is a diagram showing a body marker according to an embodiment of the present invention.

As shown in FIG. 7, the ultrasound diagnostic device 100 may generate a body marker by using an image of a testee and information regarding location of a probe.

For example, if a user acquires an ultrasound image with respect to heart, the ultrasound diagnostic device 100 may generate an image of a testee in which a probe is located at a location ① as a body marker. If a user acquires an ultrasond image with repsect to thyroid, the ultrasound diagnostic device 100 may generate an image of a testee in which the probe is located at a location ② as a body marker.

A method according to an embodiment of the present invention may be embodied in the form of program commands that may be carried out by various computer devices and may be stored in a computer readable recording medium. The computer readable recording medium may include program commands, data files, data structures, or combinations thereof. The program commands stored in the computer readable recording medium may be either exclusively designed and configured for the present invention or known in the art. Examples of computer readable recording media include magnetic media, such as a floppy disk and a magnetic tape, optical media, such as a CD-ROM and a DVD, magneto-optical media, such as a floptical disk, and hardware devices exclusively configured to store and carry out program commands, such as a ROM, a RAM, and a flash memory. Examples of the program commands include not only machine languages generated by compilers, but also high-level languages that may be carried out by a computer via an interpreter.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A method of providing a body marker carried out by a device according to any of claims 6 - 10, the method comprising:
when an ultrasound image of a target body is acquired by using a probe, acquiring an image of a testee including information regarding a location of the probe on the testee by using an imaging camera;
extracting frame information regarding bones or pose of the testee and information regarding the location of the probe from the image of the testee;
generating a body marker indicating a location of the target body based on the frame information and the information regarding the location of the probe; and
mapping the body marker to the ultrasound image of the target body and storing a mapped result,
wherein the generating the body marker comprises:
comparing the image of the testee to a pre-stored body model based on the frame information and modifying the pre-stored body model to be fitted to the image of the testee; and
generating the body marker indicating the location of the target body based on the modified body model and the information regarding the location of the probe.

2. The method of claim 1, wherein the acquiring the image of the testee comprises acquiring the image of the testee by using the imaging camera which comprises at least one of an image sensor (111), an infrared ray (IR) sensor (113), and a thermal image sensor (115).

3. The method of claim 1, wherein the generating the body marker comprises generating the body marker in the form of at least one of a 2-dimensional image, a 3-dimensional image, and a 4-dimensional image.

4. The method of claim 1, further comprising displaying the stored ultrasound image and the body marker.

5. The method of claim 1, further comprising:
extracting the target body corresponding to the information regarding the location of the probe; and
setting functions for diagnosing the target body to the probe.

6. An ultrasound diagnostic device (100) comprising:
a testee image acquiring unit (110), which, when an ultrasound image of a target body is acquired by using a probe, acquires an image of a testee including information regarding a location of the probe using an imaging camera;
a body marker generating unit (120), which extracts frame information regarding bones or pose of the testee and information regarding a location of the probe from the image of the testee and generates a body marker indicating location of the target body based on the frame information and the information regarding the location of the probe;
a storage unit (130), which maps the body marker to the ultrasound image of the target body and stores a mapped result; and
a control unit (140), which controls the testee image acquiring unit (110), the body marker generating unit (120), and the storage unit (130),
wherein the body marker generating unit (120) compares the image of the testee to a pre-stored body model based on the frame information, modifies the pre-stored body model to be fitted to the image of the testee, and generates a body marker indicating a location of the target body based on the modified body model and the information regarding the location of the probe.

7. The ultrasound diagnostic device (100) of claim 6, wherein the testee image acquiring unit (110) acquires the image of the testee by using the imaging camera which comprises at least one of an image sensor (111), an infrared ray (IR) sensor (113), and a thermal image sensor (115).

8. The ultrasound diagnostic device (100) of claim 6, wherein the body marker generating unit (120) generates the body marker in the form of at least one of a 2-dimensional image, a 3-dimensional image, and a 4-dimensional image.

9. The ultrasound diagnostic device (100) of claim 6, further comprising a display unit (150), which displays the stored ultrasound image and the body marker.

10. The ultrasound diagnostic device (100) of claim 6, wherein the control unit (140) extracts the target body corresponding to the information regarding the location of the probe and sets functions for diagnosing the target body to the probe.

11. A computer readable recording medium having recorded thereon a computer program for implementing the method of any of claims 1 through 5.

## Patentansprüche

1. Verfahren zur Erzeugung eines Körpermarkers, das mit einer Vorrichtung nach einem der Ansprüche 6 - 10 durchgeführt wird, wobei das Verfahren Folgendes aufweist:
wenn ein Ultraschallbild eines Zielkörpers unter Verwendung einer Sonde erfasst wird, Erlangen eines Bildes einer Testperson, das Informationen bezüglich einer Position der Sonde auf der Testperson enthält, unter Verwendung einer Abbildungskamera;
Extrahieren von Bildinformationen bezüglich der Knochen oder der Haltung der Testperson und Informationen bezüglich der Position der Sonde aus dem Bild der Testperson;
Erzeugen eines Körpermarkers, der eine Position des Zielkörpers basierend auf den Bildinformationen und den Informationen bezüglich der Position der Sonde anzeigt; und
Erfassen des Körpermarkers auf dem Ultraschallbild des Zielkörpers und Speichern eines erfassten Ergebnisses,
wobei das Erzeugen des Körpermarkers Folgendes aufweist:
Vergleichen des Bildes der Testperson mit einem vorgespeicherten Körpermodell basierend auf den Bildinformationen und Modifizieren des vorgespeicherten Körpermodells, um es an das Bild der Testperson anzupassen; und
Erzeugen des Körpermarkers, der die Position des Zielkörpers basierend auf dem modifizierten Körpermodell und den Informationen bezüglich der Position der Sonde anzeigt.

2. Verfahren nach Anspruch 1, wobei das Erlangen des Bildes der Testperson das Erlangen des Bildes der Testperson unter Verwendung der Abbildungskamera aufweist, die wenigstens eines eines Bildsensors (111), eines Infrarotstrahlensensors (113) und eines Wärmebildsensors (115) aufweist.

3. Verfahren nach Anspruch 1, wobei das Erzeugen des Körpermarkers das Erzeugen des Körpermarkers in Form eines 2-dimensionalen Bildes, eines 3-dimensionalen Bildes und/oder eines 4-dimensionalen Bildes umfasst.

4. Verfahren nach Anspruch 1, welches des Weiteren das Anzeigen des gespeicherten Ultraschallbildes und des Körpermarkers aufweist.

5. Verfahren nach Anspruch 1, welches des Weiteren Folgendes aufweist:
Extrahieren des Zielkörpers entsprechend den Informationen über die Position der Sonde; und
Einstellen von Funktionen zur Diagnose des Zielkörpers an der Sonde.

6. Ultraschall-Diagnosevorrichtung (100), welche Folgendes aufweist:
eine Testpersonenbild-Erlangungseinheit (110), die, wenn ein Ultraschallbild eines Zielkörpers unter Verwendung einer Sonde erlangt wird, ein Bild einer Testperson erlangt, das Informationen bezüglich einer Position der Sonde unter Verwendung einer Abbildungskamera enthält;
eine Körpermarker-Erzeugungseinheit (120), die Bildinformationen bezüglich der Knochen oder der Haltung der Testperson und Informationen bezüglich einer Position der Sonde aus dem Bild der Testperson extrahiert und einen Körpermarker erzeugt, der eine Position des Zielkörpers basierend auf den Bildinformationen und den Informationen bezüglich der Position der Sonde anzeigt;
eine Speichereinheit (130), die den Körpermarker auf dem Ultraschallbild des Zielkörpers erfasst und ein erfasstes Ergebnis speichert; und
eine Steuereinheit (140), die die Testpersonenbild-Erlangungseinheit (110), die Körpermarker-Erzeugungseinheit (120) und die Speichereinheit (130) steuert,
wobei die Körpermarker-Erzeugungseinheit (120) das Bild der Testperson mit einem vorgespeicherten Körpermodell basierend auf den Bildinformationen vergleicht, das vorgespeicherte Körpermodell modifiziert, um es an das Bild der Testperson anzupassen, und einen Körpermarker erzeugt, der eine Position des Zielkörpers basierend auf dem modifizierten Körpermodell und den Informationen bezüglich der Position der Sonde anzeigt.

7. Ultraschall-Diagnosevorrichtung (100) nach Anspruch 6, wobei die Testpersonenbild-Erlangungseinheit (110) das Bild der Testperson unter Verwendung der Abbildungskamera, die wenigstens eines eines Bildsensors (111), eines Infrarotstrahlensensors (113) und eines Wärmebildsensors (115) aufweist, erlangt.

8. Ultraschall-Diagnosevorrichtung (100) nach Anspruch 6, wobei die Körpermarkierungs-Erzeugungseinheit (120) die Körpermarkierung 2-dimensionalen Bildes, eines 3-dimensionalen Bildes und/oder eines 4-dimensionalen Bildes erzeugt.

9. Ultraschall-Diagnosevorrichtung (100) nach Anspruch 6, welche des Weiteren eine Anzeigeeinheit (150) aufweist, die das gespeicherte Ultraschallbild und den Körpermarker anzeigt.

10. Ultraschall-Diagnosevorrichtung (100) nach Anspruch 6, wobei die Steuereinheit (140) den Zielkörper entsprechend den Informationen über die Position der Sonde extrahiert und Funktionen zur Diagnose des Zielkörpers an der Sonde einstellt.

11. Computerlesbares Aufzeichnungsmedium, auf dem ein Computerprogramm zur Durchführung des Verfahrens nach einem der Ansprüche 1 bis 5 aufgezeichnet ist.

## Revendications

1. Procédé de fourniture d'un marqueur corporel réalisé par un dispositif selon l'une quelconque des revendications 6 à 10, le procédé comprenant les étapes consistant à :
lorsqu'une image ultrasonore d'un corps cible est acquise à l'aide d'une sonde, acquérir une image d'un sujet testé comprenant des informations concernant un emplacement de la sonde sur le sujet testé à l'aide d'un dispositif de prise de vues d'imagerie ;
extraire des informations de trame concernant des os ou la posture du sujet testé et des informations concernant l'emplacement de la sonde à partir de l'image du sujet testé ;
générer un marqueur corporel indiquant un emplacement du corps cible sur la base des informations de trame et des informations concernant l'emplacement de la sonde ; et
mapper le marqueur corporel sur l'image ultrasonore du corps cible et stocker un résultat mappé,
dans lequel la génération du marqueur corporel comprend les étapes consistant à :
comparer l'image du sujet testé à un modèle corporel pré-stocké sur la base des informations de trame et modifier le modèle corporel pré-stocké pour l'ajuster à l'image du sujet testé ; et
générer le marqueur corporel indiquant l'emplacement du corps cible sur la base du modèle corporel modifié et des informations concernant l'emplacement de la sonde.

2. Procédé selon la revendication 1, dans lequel l'acquisition de l'image du sujet testé comprend l'acquisition de l'image du sujet testé en utilisant le dispositif de prise de vues d'imagerie qui comprend au moins l'un parmi un capteur d'image (111), un capteur (113) à rayon infrarouge (IR), et un capteur d'image thermique (115).

3. Procédé selon la revendication 1, dans lequel la génération du marqueur corporel comprend la génération du marqueur corporel sous la forme d'au moins l'une parmi une image bidimensionnelle, une image tridimensionnelle, et une image quadridimensionnelle.

4. Procédé selon la revendication 1, comprenant en outre un l'affichage de l'image ultrasonore stockée et du marqueur corporel.

5. Procédé selon la revendication 1, comprenant en outre :
l'extraction du corps cible correspondant aux informations concernant l'emplacement de la sonde ; et
l'affectation de fonctions pour diagnostiquer le corps cible à la sonde.

6. Dispositif de diagnostic à ultrasons (100) comprenant :
une unité d'acquisition d'image de sujet testé (110) qui, lorsqu'une image ultrasonore d'un corps cible est acquise à l'aide d'une sonde, acquiert une image d'un sujet testé comprenant des informations concernant un emplacement de la sonde à l'aide d'un dispositif de prise de vues d'imagerie ;
une unité de génération de marqueur corporel (120), qui extrait des informations de trame concernant des os ou la posture du sujet testé et des informations concernant un emplacement de la sonde à partir de l'image du sujet testé et génère un marqueur corporel indiquant l'emplacement du corps cible sur la base des informations de trame et des informations concernant l'emplacement de la sonde ;
une unité de stockage (130), qui mappe le marqueur corporel sur l'image ultrasonore du corps cible et stocke un résultat mappé ; et
une unité de commande (140), qui commande l'unité d'acquisition d'image de sujet testé (110), l'unité de génération de marqueur corporel (120), et l'unité de stockage (130),
dans lequel l'unité de génération de marqueur corporel (120) compare l'image du sujet testé à un modèle corporel pré-stocké sur la base des informations de trame, modifie le modèle corporel pré-stocké pour l'ajuster à l'image du sujet testé, et génère un marqueur corporel indiquant un emplacement du corps cible sur la base du modèle corporel modifié et des informations concernant l'emplacement de la sonde.

7. Dispositif de diagnostic à ultrasons (100) selon la revendication 6, dans lequel l'unité d'acquisition d'image de sujet testé (110) acquiert l'image du sujet testé en utilisant le dispositif de prise de vues d'imagerie qui comprend au moins l'un parmi un capteur d'image (111), un capteur (113) à rayon infrarouge (IR), et un capteur d'image thermique (115).

8. Dispositif de diagnostic à ultrasons (100) selon la revendication 6, dans lequel l'unité de génération de marqueur corporel (120) génère le marqueur corporel sous la forme d'au moins l'une parmi une image bidimensionnelle, une image tridimensionnelle, et une image quadridimensionnelle.

9. Dispositif de diagnostic à ultrasons (100) selon la revendication 6, comprenant en outre une unité d'affichage (150), qui affiche l'image ultrasonore stockée et le marqueur corporel.

10. Dispositif de diagnostic à ultrasons (100) selon la revendication 6, dans lequel l'unité de commande (140) extrait le corps cible correspondant aux informations concernant l'emplacement de la sonde et affecte des fonctions pour diagnostiquer le corps cible à la sonde.

11. Support d'enregistrement lisible par ordinateur sur lequel est enregistré un programme informatique destiné à mettre en œuvre le procédé selon l'une quelconque des revendications 1 à 5.
